# FASCICULE DE BREVET EUROPEEN

(11) **EP 2 203 405 B1**
(45) Date de publication et mention de la délivrance du brevet: **17.08.2011**
(21) Numéro de dépôt: 08832893.5
(22) Date de dépôt: 25.09.2008
(51) Int. Cl.: C07C 51/44, C07C 53/18, C07C 53/15, B01D 3/34, C02F 1/04

(54) **PROCEDE DE RECUPERATION D'ACIDES FLUOROCARBOXYLIQUES**
VERFAHREN ZUR RÜCKGEWINNUNG VON FLUORCARBONSÄUREN
METHOD FOR RECOVERING FLUOROCARBOXYLIC ACIDS

(30) Priorité: 28.09.2007 FR 0706829
(43) Date de publication de la demande: 07.07.2010
(73) Titulaire: Rhodia Opérations, 93306 Aubervilliers (FR)
(72) Inventeur: MERCIER, Claude, F-84150 Jonquieres (FR); METZ, François, F-69540 Irigny (FR)
(74) Mandataire: Dutruc-Rosset, Marie-Claude
(86) Numéro de dépôt international: PCT/EP2008/062852
(87) Numéro de publication internationale: WO 2009/040398

(56) Documents cités:
- RU-C1- 2 151 741
- ANONYME: "Image:Simple distillation apparatus.png" WIKIPEDIA, THE FREE ENCYCLOPEDIA, [Online] 15 août 2007 (2007-08-15), XP002476613 Extrait de l'Internet: URL:http://en.wikipedia.org/wiki/Image:Sim ple_distillation_apparatus.png> [extrait le 2008-04-15]

## Description

La présente invention a pour objet un procédé de récupération d'acides fluorocarboxyliques à partir de solutions aqueuses contenant ces acides. L'invention concerne plus particulièrement la récupération d'acides fluorocarboxyliques formant un azéotrope avec l'eau, par contact avec un acide fort.

L'industrie chimique utilise, dans de nombreux domaines d'activités, des acides fluorocarboxyliques, lesquels sont pour la plupart solubles dans l'eau et sont souvent présents en quantités plus ou moins importantes dans les rejets industriels, notamment les eaux usées.

En raison des coûts de ces acides fluorocarboxyliques, il peut s'avérer utile et rentable de traiter les effluents aqueux contenant de tels acides, afin des les récupérer.

En outre, la présence d'acides fluorocarboxyliques dans les rejets industriels aqueux peut être nuisible à l'environnement et les normes environnementales devenant de plus en plus sévères, la récupération des acides fluorocarboxyliques devient maintenant un problème pour lequel aucune solution acceptable n'est encore disponible sur le plan industriel.

Un moyen simple et efficace pourrait consister en la distillation du résidu aqueux comprenant le ou les acide(s) fluorocarboxylique(s). Cependant, ces acides forment généralement des azéotropes avec l'eau, rendant leur distillation jusqu'à un degré de pureté satisfaisant impossible. Par exemple, l'acide trifluoroacétique (TFA) forme un azéotrope avec l'eau, dont le point d'ébullition est d'environ 103°C. Cette température, très proche de celle du point d'ébullition de l'eau, rend la distillation simple TFA/eau encore moins efficace, et non utilisable.

Ainsi, un objectif de l'invention est de proposer un procédé simple et efficace permettant la récupération d'acides fluorocarboxyliques à partir d'effluents aqueux, avec un rendement et un degré de pureté satisfaisants et économiquement rentables, notamment sur le plan industriel.

D'autres objectifs apparaîtront dans la suite de l'exposé de la présente invention.

Comme indiqué précédemment, un procédé classique de distillation ne permet pas la récupération d'un acide fluorocarboxylique de pureté élevée, en raison de l'azéotrope que forme ledit acide avec l'eau. Les inventeurs ont maintenant découvert un procédé, objet de l'invention, qui permet la récupération d'acides fluorocarboxyliques présents dans des effluents (ou plus simplement des solutions) aqueuses.

Ainsi, la présente invention concerne tout d'abord un procédé de récupération d'acide(s) fluorocarboxylique(s) à partir d'un effluent aqueux, caractérisé par le fait qu'il comprend les étapes suivantes :
a) mise en contact dudit effluent aqueux comprenant au moins un acide fluorocarboxylique avec au moins un acide fort qui possède un pKa dans l'eau inférieur à 0,1, à 20°C ;
b) distillation du mélange obtenu à l'étape a) ;
c) récupération du distillat de l'étape b) consistant essentiellement en ledit acide fluorocarboxylique.

Il a en effet été découvert que l'acide fort en mélange avec l'effluent aqueux permet de « casser » l'azéotrope acide fluorocarboxylique/eau, rendant possible la distillation dudit acide fluorocarboxylique, de manière efficace et rentable du point de vue du rendement et de la pureté.

Par effluent aqueux, on entend tout résidu, solution, rejet, industriel ou autre comprenant de 5 % à 70 % en poids, de préférence de 10 à 50 % en poids d'au moins un acide fluorocarboxylique par rapport au poids total de l'effluent à traiter.
Le restant de l'effluent est constitué d'eau mais peut en outre comprendre de 0 % à 20 % en poids, de préférence de 0 % à 15 % en poids d'impuretés, par rapport au poids d'acide(s) fluorocarboxylique(s). Ces impuretés peuvent être de toute nature, et sont généralement constituées d'impuretés résiduaires de procédés de préparations de composés chimiques, telles que notamment solvants organiques, réactifs en excès, intermédiaires de synthèse, sous-produits réactionnels, et autres.
Ainsi, la quantité d'eau présente dans les effluents à traiter est généralement comprise entre 15 % et 95 % en poids, de préférence de 40 % à 80 % en poids, par rapport au poids total de l'effluent.

Les acides fluorocarboxyliques qui peuvent être récupérés selon le procédé de l'invention sont plus particulièrement les acides fluorocarboxyliques aliphatiques, qui forment un azéotrope avec l'eau.

Ces acides carboxyliques fluorés possèdent préférentiellement une chaîne aliphatique, linéaire ou ramifiée, comportant de 1 à 10 atomes de carbone au total et répondent à la formule générale (AF) suivante :

CₙFₘHₚ-COOH (AF)

dans laquelle :
- n est un nombre entier, tel que 1 ≤ n ≤ 9 ;
- m est un nombre entier, tel que 1 ≤ m ≤ 2n +1 ; et
- p est un nombre entier, tel que 0 ≤ p ≤ 2n, avec p + m = 2n +1.

Selon un mode de réalisation préféré de l'invention, les acides fluorocarboxyliques sont des acides perfluorocarboxyliques, c'est-à-dire des acides de formule (AF) pour lesquels m = 2n + 1 et p = 0. Selon un autre mode de réalisation, les acides fluorocarboxyliques sont des acides carboxyliques fluorés dans lesquels la totalité des atomes de fluor sont portés exclusivement par l'atome de carbone en position ω par rapport au groupement carboxylique (COOH), en particulier les acides de formule F₃C-(-CH₂-)ₙ₋₁ -COOH, où n est tel que défini dans la formule (AF) précédente.

Il est à noter les acides fluorocarboxyliques peuvent également comprendre des atomes d'halogène autres que le fluor, tels que le chlore ou le brome.

Des exemples d'acides fluorocarboxyliques pouvant être récupérés par le procédé de l'invention sont l'acide difluoroacétique (ou DFA, F₂HC-COOH), l'acide chlorodifluoroacétique (ou CDFA, CIF₂C-COOH), l'acide trifluoroacétique (ou TFA, F₃C-COOH), l'acide 3,3,3-trifluoropriopionique (F₃C-CH₂-COOH), l'acide pentafluoropropionique (F₅C₂-COOH), l'acide heptafluorobutyrique (F₇C₃-COOH), l'acide perfluoropentanoïque (F₉C₄-COOH), l'acide perfluorohexanoïque (F₁₁C₅-COOH), l'acide perfluoroheptanoïque (F₁₃C₆-COOH), et l'acide perfluorooctanoïque (F₁₅C₇-COOH).
L'acide trifluoroacétique (TFA), qui forme naturellement un azéotrope positif (point d'ébullition : 103°C) avec l'eau est tout particulièrement préféré dans le procédé de la présente invention.

Il doit être entendu que l'effluent aqueux mis en oeuvre dans le procédé peut comprendre un ou plusieurs acides fluorocarboxyliques, de préférence un seul, deux ou trois, de préférence encore l'effluent ne comprend qu'un seul acide fluorocarboxylique, de manière tout à fait préférée, l'effluent comprend uniquement le TFA.

L'invention n'exclut pas non plus, le cas où l'acide fluorocarboxylique provient de l'hydrolyse de l'anhydride d'acide correspondant.

Dans le procédé de l'invention, l'acide fort peut être ajouté à l'effluent aqueux, ou bien l'effluent aqueux peut être ajouté à l'acide fort.

L'acide fort qui est ainsi mélangé à l'effluent aqueux à traiter a pour effet de « piéger » l'eau dudit effluent aqueux, et de « casser » l'azéotrope eau/acide fluorocarboxylique.

Les acides forts sont de préférence des acides forts protoniques, c'est-à-dire susceptibles de libérer au moins un atome d'hydrogène. Les acides forts protoniques d'intérêt pour le procédé de l'invention possèdent généralement un pKa dans l'eau inférieur à 0,1, de préférence inférieur à -1, mesuré à 20°C. Ils doivent être inertes par rapport à l'acide fluorocarboxylique à récupérer.

Des exemples d'acides forts qui peuvent être utilisés dans le procédé de la présente invention comprennent l'acide sulfurique, les oléums (à diverses concentrations, par exemple 20 %, 30 %, 40 %), l'acide chlorhydrique à l'état liquide ou gazeux (chlorure d'hydrogène), l'acide phosphorique, l'acide chlorosulfurique, l'acide fluorosulfurique, l'acide perchlorique, et les acides sulfoniques comme par exemple, l'acide méthanesulfonique, l'acide trifluorométhanesulfonique, l'acide toluènesulfonique, ou encore l'acide phénolsulfonique.

L'acide nitrique n'est toutefois pas préféré, en raison de son très fort pouvoir oxydant et de sa possibilité à réagir avec l'acide fluorocarboxylique à récupérer.

Il est bien entendu également possible d'utiliser des acides forts solides, notamment des acides supportés, par exemple les résines sulfoniques, et plus particulièrement les résines commercialisées sous différentes dénominations commerciales, parmi lesquelles on peut citer entre autres, les résines Temex^{®} 50, Amberlyst^{®} 15, Amberlyst^{®} 35, Amberlyst^{®} 36, Dowex^{®} 50W.

Les résines précitées sont constituées d'un squelette polystyrénique qui porte des groupes fonctionnels qui sont des groupes sulfoniques. Le squelette polystyrénique est obtenu par polymérisation du styrène et du divinylbenzène, sous l'influence d'un catalyseur d'activation, le plus souvent un peroxyde organique, ce qui conduit à un polystyrène réticulé qui est ensuite traité par de l'acide sulfurique ou sulfochlorique concentré conduisant à un copolymère styrène-divinylbenzène sulfoné.

Il est également possible de faire appel à des résines sulfoniques qui sont des copolymères phénol-formol et qui portent sur le noyau aromatique un groupe méthylènesulfonique, par exemple la résine commercialisée sous la dénomination Duolite^{®} ARC 9359.

D'autres résines disponibles sur le marché conviennent également et l'on peut citer les résines perfluorées porteuses de groupes sulfoniques et plus particulièrement le Nafion^{®} qui est un copolymère de tétrafluoroéthylène et de perfluoro-[2-(fluorosulfonyléthoxy)propyl]vinyléther.

Des mélanges de deux ou plusieurs des acides cités précédemment, qu'ils soient liquides, gazeux ou solides (supportés) peuvent être utilisés, en toutes proportions. En règle générale, l'acide sulfurique, notamment l'acide sulfurique à 98 % en poids, a montré de bons résultats, notamment lors de la récupération de TFA.

La quantité d'acide(s) mélangé(s) à l'effluent à traiter doit être suffisante pour « casser » l'azéotrope eau/acide fluorocarboxylique. Un excès d'acide n'est toutefois pas nuisible, mais est avantageusement évité, pour des raisons évidentes de coût de procédé de récupération.

Aussi, en règle générale, le ratio pondéral acide(s)/eau contenue dans l'effluent, est compris entre 1 et 10, ce ratio étant de préférence compris entre 1,5 et 3.

L'ajout de l'acide fort dans l'effluent aqueux à traiter peut être effectué en une fois, ou par portions, par exemple au goutte-à-goutte, en contrôlant l'exothermie résultant de cette addition d'acide fort au milieu aqueux.

De même, l'effluent aqueux peut être ajouté à l'acide fort en une fois ou par portions, avec les précautions qui s'imposent lors de l'addition d'eau dans un acide fort.

Bien que cela soit envisageable, il n'est pas nécessaire de refroidir le milieu, lors du mélange acide fort/effluent aqueux. En effet, lorsqu'elle est contrôlée, l'exothermie peut avantageusement être utilisée pour chauffer l'ensemble du milieu réactionnel, voire l'amener à ébullition, en vue de l'étape de distillation ultérieure.

Le mélange de l'acide fort et de l'effluent à traiter peut être effectué directement dans le réacteur où est conduite la distillation. Selon un autre aspect, il peut être envisagé de réaliser une ou plusieurs campagnes de mélange acide fort / effluent à traiter, puis de charger le réacteur directement avec le ou les mélanges ainsi préparés.

L'étape de distillation du mélange acide fort / effluent à traiter est conduite de manière classique, mettant en oeuvre un appareillage et des conditions connues de l'homme du métier.

Il peut être envisagé de conduire une première distillation, puis d'ajouter une quantité d'acide fort complémentaire et de terminer ensuite la distillation.

Il peut également être envisagé d'effectuer l'étape de distillation par lot (batch) ou en continu. Pour un procédé en continu, il peut être envisagé d'ajouter en continu l'acide fort dans l'effluent à traiter qui forme le pied de distillation, ou bien d'ajouter en continu l'effluent à traiter dans l'acide fort qui forme le pied de distillation, ou bien encore d'ajouter en continu, dans le réacteur, le mélange préalablement préparé acide fort / effluent à traiter.

Le dispositif de distillation est de tout type connu en soi et comprend par exemple un réacteur (bouilleur) contenant l'effluent à traiter et l'acide fort, ledit réacteur étant muni de moyens de chauffage, par exemple bain d'huile, de sable, double-enveloppe et autres moyens connus permettant de porter à ébullition (conditions de reflux) le mélange contenu dans le bouilleur.

Le bouilleur est également muni de moyens permettant la distillation et la condensation des vapeurs issues du mélange réactionnel porté au reflux. Ces moyens peuvent prendre toutes formes connues, telles que colonne à distiller, à plateaux, à garnissage et autres, condenseur, évaporateur, de type classique, par exemple à circulation de liquide de refroidissement, ou encore évaporateur à couche mince (« Thin Film Evaporator » en langue anglaise) ou à film raclé (« Wiped Film Evaporator » en langue anglaise) tel que commercialisé par exemple par la société Luwa.

En outre le dispositif de distillation comprend, éventuellement mais avantageusement, des moyens permettant l'agitation du mélange à distiller. Le dispositif de distillation comprend également des moyens de mesure de la température, avantageusement pour la mesure de la température du mélange à distiller et de la température du distillat. Ces moyens de mesure de la température sont connus de l'homme du métier et peuvent être à lecture optique, à enregistrement, voire pilotés, notamment par programme d'ordinateur.

Comme indiqué plus haut, la distillation du mélange effluent aqueux/acide fort est réalisée de manière classique, à reflux du mélange. La distillation est généralement conduite à pression atmosphérique, mais il peut être envisagé d'opérer sous vide partiel ou poussé (par exemple entre 750 mm de mercure et 50 mm de mercure, soit environ entre 1000 hPa et 67 hPa), ou encore sous pression. Il peut également être envisagé de travailler sous atmosphère inerte, azote ou argon, par exemple.

Pour des raisons économiques et de commodité, on préfère toutefois réaliser l'étape de distillation à pression atmosphérique, sans utilisation de gaz inerte.

La distillation spécifique de l'acide fluorocarboxylique est rendue possible par la présence de l'acide fort qui permet de « casser » l'azéotrope qui se forme naturellement entre l'acide fluorocarboxylique et l'eau contenue dans l'effluent aqueux.

Ainsi, lors de la distillation, l'acide (ou les acides) fluorocarboxylique(s) distillent à son(leur) point d'ébullition propre, permettant ainsi de récupérer ledit acide (ou acides) fluorocarboxylique(s) en tête du dispositif de distillation (généralement en tête de colonne) avec un degré de pureté élevé. Dans le cas préféré de l'acide trifluoroacétique (TFA), il est ainsi possible de distiller cet acide à son point d'ébullition propre, soit environ 72°C, à pression atmosphérique. Dans le cas des acides fluorocarboxyliques dont le point d'ébullition propre est supérieur à celui de l'eau (100°C à pression atmosphérique), l'eau est distillée en premier lieu, puis l'acide fluorocarboxylique de point d'ébullition propre est supérieur à celui de l'eau.

Par « degré de pureté élevé », on entend une pureté supérieure ou égale à 95 % en poids, de préférence supérieure ou égale à 99 % en poids.

Le procédé de l'invention présente également l'avantage d'être facile à mettre en oeuvre et de nécessiter un faible investissement, tout en offrant un rendement de récupération d'acide fluorocarboxylique acceptable, voire satisfaisant.

Ainsi, le rendement de récupération en acides fluorocarboxyliques est généralement supérieur à 50 % en poids, de préférence supérieur à 75 % en poids, et peuvent même aller jusqu'à des valeurs supérieures à 90 % en poids, voire supérieures à 95 % en poids.

À l'issue de l'étape de distillation, le réacteur (bouilleur) contient un résidu comprenant l'acide fort ayant servi à casser l'azéotrope, et éventuellement de l'eau, si celle-ci n'a pas été distillée avant ou après la distillation de l'acide fluorocarboxylique. Il est bien entendu que l'acide fort résiduel peut avantageusement être recyclé et engagé à nouveau dans un nouveau procédé de récupération d'acide fluorocarboxylique.

Selon un autre aspect, l'invention concerne un dispositif de distillation, ledit dispositif comprenant :
- un réacteur (bouilleur) contenant un milieu réactionnel comprenant un effluent aqueux comprenant de 5 % à 70 % en poids, de préférence de 10 à 50 % en poids d'au moins un acide fluorocarboxylique par rapport au poids total dudit effluent, et au moins un acide fort, le ratio pondéral acide(s)/eau contenue dans l'effluent étant compris entre 1 et 10 ;
- des moyens de chauffage ;
- de moyens de distillation et de condensation des vapeurs issues du mélange réactionnel porté au reflux ;
- des moyens de mesure et/ou de contrôle de la température, et éventuellement des moyens permettant l'agitation du mélange réactionnel, ou des moyens permettant de contrôler l'ébullition dudit mélange.

La présente invention a également pour objet une installation industrielle comprenant le dispositif de distillation précédemment défini, des moyens de stockage et/ou d'arrivées d'effluents aqueux contenant au moins un acide fluorocarboxylique et d'au moins un acide fort, tels que définis précédemment, ainsi que des moyens de récupération (et éventuellement de stockage) d'acide(s) fluorocarboxylique(s) issu(s) du dispositif de distillation, et des moyens d'évacuation (et éventuellement de stockage) des effluents aqueux et acides qui forment les résidus du dispositif de distillation.

La présente invention est maintenant illustrée aux moyens des exemples qui suivent et qui n'ont aucune vocation à limiter la portée de protection de la présente invention définie par les revendications annexées.

### EXEMPLES

On décrit ci-après le protocole opératoire qui sera repris dans les exemples.

On utilise un réacteur (verre ou inox, par exemple), équipé d'une colonne à distiller, d'un réfrigérant (0°C), d'une ampoule de coulée et d'un piège à humidité (CaCl₂). Le réacteur est placé dans un bain d'huile et muni d'un thermomètre et d'un agitateur mécanique.

Le résidu liquide industriel aqueux contenant un acide fluorocarboxylique est chargé dans le réacteur.

De l'acide sulfurique à 98 % en poids est ajouté goutte-à-goutte à l'acide fluorocarboxylique, sans chauffage.

Après addition de la totalité de l'acide sulfurique, le réacteur est chauffé au moyen du bain d'huile.

Le milieu réactionnel est porté à reflux. La température en tête de la colonne passe de la température ambiante à la température du point d'ébullition de l'acide fluorocarboxylique à récupérer.

Le reflux total est maintenu pendant une demi-heure, puis on commence à soutirer le distillat à la température de point d'ébullition de l'acide fluorocarboxylique en sommet de colonne avec un rapport de reflux compris entre 1 et 5, de préférence d'au moins 3 à 4.

Lorsque la quasi totalité de l'acide fluorocarboxylique est distillée, on augmente la température à l'intérieur du réacteur, température que l'on maintient jusqu'à diminution de la température des vapeurs. On arrête alors le soutirage de l'acide fluorocarboxylique.

Le réacteur est refroidi à température ambiante et le résidu est versé dans un fût de récupération des effluents.

### Exemple 1 :

On charge 250 g d'un rejet liquide industriel aqueux contenant 30 % en poids d'acide trifluoroacétique (TFA), 2,4 % en poids de toluène, et 1,8 % en poids de pyridine, dans un réacteur de 500 mL, équipé d'un agitateur magnétique, de moyens de chauffage, d'un dispositif de distillation et d'un thermomètre.

On chauffe le réacteur jusqu'au reflux total pendant au moins une demi-heure (température en pied : 102°C ; température en tête : 100°C), puis on ajoute goutte-à-goutte 300 g d'acide sulfurique.

La température des vapeurs chute à 72°C et on commence à soutirer le distillat à 72°C en tête de colonne.

Lorsque la température en tête augmente (disparition du reflux), on stoppe la récupération du distillat.

On récupère ainsi 55 g d'acide trifluoroacétique (rendement 73 %) présentant un degré de pureté supérieur à 99,8 %, et un taux d'humidité de 357 ppm mesuré par la méthode de Karl-Fischer.

### Exemple 2 :

On réalise un deuxième essai, selon le mode opératoire décrit dans l'exemple 1, en mélangeant 215 g d'une solution aqueuse à 29 % en poids d'acide trifluoroacétique avec 322 g d'acide sulfurique à 98 % en poids.

On récupère 45,15 g d'acide trifluoroacétique (rendement 73 %) présentant un degré de pureté supérieur à 99,8 %, et un taux d'humidité de 403 ppm mesuré par la méthode de Karl-Fischer.

### Exemple 3:

On réalise un troisième essai, selon le mode opératoire décrit dans l'exemple 1, en mélangeant 200 g d'une solution aqueuse à 30 % en poids d'acide trifluoroacétique, contenant 10,65 g de toluène, avec 241 g d'acide sulfurique à 98 % en poids.

On récupère 47,7 g d'acide trifluoroacétique (rendement 79,2 %) présentant un degré de pureté supérieur à 99,8 %, et un taux d'humidité de 260 ppm mesuré par la méthode de Karl-Fischer.

### Exemple 4 :

On utilise un effluent aqueux à 30 % en poids de TFA, et de l'acide sulfurique à 98 % (Sinopharm, grade A.R).

On charge 244,1 g d'acide sulfurique dans un réacteur de 500 mL, muni d'une colonne de distillation, puis on ajoute précautionneusement, au goutte-à-goutte, 204,7 g d'effluent aqueux contenant 30 % en poids d'acide trifluoroacétique, tout en commençant à chauffer l'ensemble.

Le reflux apparaît à 72°C en tête de colonne, alors que la température du réacteur diminue jusqu'à 126°C.

La température en tête de colonne est stabilisée à 72°C et on commence à récupérer le TFA pur, et la température en pied augmente lentement à 138°C, température que l'on maintient jusqu'à l'arrêt de la distillation de TFA.

On récupère 45,1 g de TFA (rendement 73,7 %) présentant un degré de pureté supérieur à 99,8 %.

### Exemples 5 à 14

Les essais correspondants sont réalisés selon le mode opératoire décrit dans l'exemple 1, en mélangeant 215 g d'une solution aqueuse à x % en poids d'acide carboxylique perhalogéné avec y g d'acide minéral à z % en poids.

Les résultats obtenus sont consignés dans le tableau (I).

**Tableau (I)**

| Ex | Acide perhalogéné | Concentration % poids (x) | Acide minéral (z) | Poids Acide minéral g (y) | Taux de recupération (%) | Pureté % | Taux humidité % |
|---|---|---|---|---|---|---|---|
| 5 | CF₃CO₂H | 10 | H₂SO₄ | 355 | 39,1 | 98 | 1 |
| | | | (98%) | | | | |
| 6 | | 28 | H₂SO₄ | 158 | 38,3 | 99 | - |
| | | | (98%) | | | | |
| 7 | | 28 | H₂SO₄ | 474 | 93,6 | 99,6 | 0,05 |
| | | | (98 %) | | | | |
| 8 | | 50 | H₂SO₄ | 197 | 91,3 | 99,8 | 0,1 |
| | | | (98 %) | | | | |
| 9 | | 28 | PPA* | 135 | 45,4 | 99,4 | 0,1 |
| | | | (80 %) | | | | |
| 10 | | 28 | P₂O₅ | 309 | 49,1 | 98,9 | 0,67 |
| | | | (100 %) | | | | |
| 11 | CCIF₂CO₂H | 30 | H₂SO₄ | 461 | 25,8 | 92,5 | 7,5 |
| | | | (98 %) | | | | |
| 12 | CF₃CF₂CO₂H | 30 | H₂SO₄ | 276 | 54,4 | 98,8 | 38 ppm |
| | | | (98 %) | | | | |
| 13 | CF₃(CF₂)₂CO₂H | 30 | H₂SO₄ | 353 | 57 | 95 | 5 |
| | | | (98 %) | | | | |
| 14 | CF₃(CF₂)₂CO₂H | 30 | H₂SO₄ | 691 | 43 | 96,3 | 0,38 |
| | | | (98 %) | | | | |

| | | | | | | | |
|---|---|---|---|---|---|---|---|
| * PPA = acide polyphosphorique (80 % P₂O₅) | | | | | | | |

## Revendications

1. Procédé de récupération d'acide(s) fluorocarboxylique(s) à partir d'un effluent aqueux, **caractérisé par le fait qu'**il comprend les étapes suivantes :
a) mise en contact dudit effluent aqueux comprenant au moins un acide fluorocarboxylique avec au moins un acide fort qui possède un pKa dans l'eau inférieur à 0,1, à 20°C ;
b) distillation du mélange obtenu à l'étape a) ;
c) récupération du distillat de l'étape b) consistant essentiellement en ledit acide fluorocarboxylique.

2. Procédé selon la revendication 1, **caractérisé par le fait que** ledit acide fort est ajouté audit effluent aqueux.

3. Procédé selon la revendication 1, **caractérisé par le fait que** ledit effluent aqueux est ajouté audit acide fort.

4. Procédé selon l'une quelconque des revendications précédentes, **caractérisé par le fait que** l'effluent aqueux comprend de 5 % à 70 % en poids, de préférence de 10 % à 50 % en poids d'au moins un acide fluorocarboxylique par rapport au poids total dudit effluent.

5. Procédé selon l'une quelconque des revendications précédentes, **caractérisé par le fait que** l'effluent aqueux comprend de 0 % à 20 % en poids, de préférence de 0 % à 15 % en poids d'impuretés, par rapport au poids d'acide(s) fluorocarboxylique(s).

6. Procédé selon l'une quelconque des revendications précédentes, **caractérisé par le fait qu'**au moins un acide fluorocarboxylique est choisi parmi les acides fluorocarboxyliques aliphatiques formant un azéotrope avec l'eau, préférentiellement parmi ceux comportant une chaîne aliphatique, linéaire ou ramifiée, possédant de 1 à 10 atomes de carbone au total.

7. Procédé selon l'une quelconque des revendications précédentes, **caractérisé par le fait qu'**au moins un acide fluorocarboxylique est choisi parmi les acides perfluorocarboxyliques et les acides fluorocarboxyliques dont la totalité des atomes de fluor sont portés exclusivement par l'atome de carbone en position w par rapport au groupement carboxylique.

8. Procédé selon l'une quelconque des revendications précédentes, **caractérisé par le fait qu'**au moins un acide fluorocarboxylique est choisi parmi l'acide difluoroacétique, l'acide chlorodifluoroacétique, l'acide trifluoroacétique, l'acide 3,3,3-trifluoropriopionique, l'acide pentafluoropropionique, l'acide heptafluorobutyrique, l'acide perfluoropentanoïque, l'acide perfluorohexanoïque, l'acide perfluoroheptanoïque et l'acide perfluorooctanoïque.

9. Procédé selon l'une quelconque des revendications précédentes, **caractérisé par le fait qu'**au moins un acide fluorocarboxylique est l'acide trifluoroacétique.

10. Procédé selon l'une quelconque des revendications précédentes, **caractérisé par le fait que** l'acide fort possède un pKa dans l'eau inférieur à -1, à 20°C.

11. Procédé selon l'une quelconque des revendications précédentes, **caractérisé par le fait que** l'acide fort est choisi parmi l'acide sulfurique, les oléums (à diverses concentrations, 20 %, 30 % ou 40 %), l'acide chlorhydrique à l'état liquide ou gazeux, l'acide phosphorique, l'acide chlorosulfurique, l'acide fluorosulfurique, l'acide perchlorique, les acides sulfoniques, de préférence les acides méthanesulfonique, trifluorométhanesulfonique, toluènesulfonique, ou phénolsulfonique, les acides supportés, parmi lesquels les résines sulfoniques, les copolymères styrène-divinylbenzène sulfonés, les résines sulfoniques copolymères phénol-formol portant sur le noyau aromatique un groupe méthylènesulfonique, les résines perfluorées porteuses de groupes sulfoniques, les copolymères de tétrafluoroéthylène et de perfluoro-[2-(fluorosulfonyléthoxy)propyl]vinyléther, et leurs mélanges en toutes proportions de deux ou plusieurs d'entre eux.

12. Procédé selon l'une quelconque des revendications précédentes, **caractérisé par le fait que** l'acide fort est l'acide sulfurique, de préférence l'acide sulfurique à 98 % en poids.

13. Procédé selon l'une quelconque des revendications précédentes, **caractérisé par le fait que** le ratio pondéral acide(s)/eau contenue dans l'effluent, est compris entre 1 et 10, de préférence entre 1,5 et 3.

14. Procédé selon l'une quelconque des revendications précédentes, **caractérisé par le fait que** l'étape de distillation est conduite à pression atmosphérique.

15. Procédé selon l'une quelconque des revendications précédentes, **caractérisé par le fait que** l'acide fluorocarboxylique est récupéré avec un degré de pureté supérieur ou égal à 95 % en poids, de préférence supérieur ou égal à 99 % en poids.

16. Procédé selon l'une quelconque des revendications précédentes, **caractérisé par le fait que** le rendement de récupération est supérieur à 50 % en poids, de préférence supérieur à 75 % en poids, de préférence encore supérieur à 90 % en poids, voire supérieur à 95 % en poids.

17. Procédé selon l'une quelconque des revendications précédentes, **caractérisé par le fait que** l'étape de distillation est conduite par lot (batch).

18. Procédé selon l'une quelconque des revendications 1 à 16, **caractérisé par le fait que** l'étape de distillation est conduite en continu.

19. Utilisation pour la mise en oeuvre du procédé décrit dans l'une des revendications 1 à 18, d'un dispositif de distillation comprenant :
- un réacteur (bouilleur) contenant un milieu réactionnel comprenant un effluent aqueux comprenant de 5 % à 70 % en poids, de préférence de 10 à 50 % en poids d'au moins un acide fluorocarboxylique par rapport au poids total dudit effluent, et au moins un acide fort, le ratio pondéral acide(s)/eau contenue dans l'effluent étant compris entre 1 et 10 ;
- des moyens de chauffage ;
- de moyens de distillation et de condensation des vapeurs issues du mélange réactionnel porté au reflux ;
- des moyens de mesure et/ou de contrôle de la température, et éventuellement des moyens permettant l'agitation du mélange réactionnel.

## Claims

1. Method of recovery of fluorocarboxylic acid(s) from an aqueous effluent, **characterized in that** it comprises the following stages:
a) contacting said aqueous effluent comprising at least one fluorocarboxylic acid with at least one strong acid possessing a pKa in water below 0.1 at 20°C;
b) distillation of the mixture obtained in stage a);
c) recovery of the distillate from stage b) consisting essentially of said fluorocarboxylic acid.

2. Method according to Claim 1, **characterized in that** said strong acid is added to said aqueous effluent.

3. Method according to Claim 1, **characterized in that** said aqueous effluent is added to said strong acid.

4. Method according to any one of the preceding claims, **characterized in that** the aqueous effluent comprises from 5 to 70 wt.%, preferably from 10 to 50 wt.% of at least one fluorocarboxylic acid relative to the total weight of said effluent.

5. Method according to any one of the preceding claims, **characterized in that** the aqueous effluent comprises from 0 to 20 wt.%, preferably from 0 to 15 wt.% of impurities, relative to the weight of fluorocarboxylic acid(s).

6. Method according to any one of the preceding claims, **characterized in that** at least one fluorocarboxylic acid is selected from the aliphatic fluorocarboxylic acids forming an azeotrope with water, preferably from those with a linear or branched aliphatic chain, having from 1 to 10 carbon atoms in total.

7. Method according to any one of the preceding claims, **characterized in that** at least one fluorocarboxylic acid is selected from the perfluorocarboxylic acids and the fluorocarboxylic acids, in which all of the fluorine atoms are carried exclusively by the carbon atom in position ωrelative to the carboxyl group.

8. Method according to any one of the preceding claims, **characterized in that** at least one fluorocarboxylic acid is selected from difluoroacetic acid, chlorodifluoroacetic acid, trifluoroacetic acid, 3,3,3-trifluoropriopionic acid, pentafluoropropionic acid, heptafluorobutyric acid, perfluoropentanoic acid, perfluorohexanoic acid, perfluoroheptanoic acid and perfluorooctanoic acid.

9. Method according to any one of the preceding claims, **characterized in that** at least one fluorocarboxylic acid is trifluoroacetic acid.

10. Method according to any one of the preceding claims, **characterized in that** the strong acid possesses a pKa in water below -1 at 20°C.

11. Method according to any one of the preceding claims, **characterized in that** the strong acid is selected from sulfuric acid, oleums (at various concentrations, 20%, 30% or 40%), hydrochloric acid in the liquid or gaseous state, phosphoric acid, chlorosulfuric acid, fluorosulfuric acid, perchloric acid, sulfonic acids, preferably methanesulfonic, trifluoromethanesulfonic, toluenesulfonic, or phenolsulfonic acids, supported acids, including sulfonic resins, sulfonated styrene-divinylbenzene copolymers, phenol-formol copolymeric sulfonic resins bearing a methylenesulfonic group on the aromatic nucleus, perfluorinated resins bearing sulfonic groups, copolymers of tetrafluoroethylene and perfluoro-[2-(fluorosulfonylethoxy)propyl]vinylether, and mixtures thereof in all proportions of two or more of them.

12. Method according to any one of the preceding claims, **characterized in that** the strong acid is sulfuric acid, preferably sulfuric acid at 98 wt.%.

13. Method according to any one of the preceding claims, **characterized in that** the weight ratio of acid(s) to water contained in the effluent is between 1 and 10, preferably between 1.5 and 3.

14. Method according to any one of the preceding claims, **characterized in that** the distillation stage is carried out at atmospheric pressure.

15. Method according to any one of the preceding claims, **characterized in that** the fluorocarboxylic acid is recovered with a degree of purity greater than or equal to 95 wt.%, preferably greater than or equal to 99 wt.%.

16. Method according to any one of the preceding claims, **characterized in that** the recovery yield is greater than 50 wt.%, preferably greater than 75 wt.%, more preferably greater than 90 wt.%, or even greater than 95 wt.%.

17. Method according to any one of the preceding claims, **characterized in that** the distillation stage is carried out as a batch operation.

18. Method according to any one of Claims 1 to 16, **characterized in that** the distillation stage is carried out continuously.

19. Use, for application of the method described in one of Claims 1 to 18, of a distillation device comprising:
- a reactor (a still) containing a reaction mixture comprising an aqueous effluent comprising from 5 to 70 wit.%, preferably from 10 to 50 wt.% of at least one fluorocarboxylic acid relative to the total weight of said effluent, and at least one strong acid, the weight ratio of acid(s) to water contained in the effluent being between 1 and 10;
- heating means;
- means for distillation and condensation of the vapors coming from the reaction mixture under reflux;
- means for temperature measurement and/or control, and optionally means for stirring the reaction mixture.

## Patentansprüche

1. Verfahren zur Rückgewinnung von Fluorcarbonsäure(n) aus einem wasserhaltigen Stoffstrom, **dadurch gekennzeichnet, dass** es die folgenden Schritte umfasst:
a) Inkontaktbringen des wasserhaltigen Stoffstroms, der mindestens eine Fluorcarbonsäure enthält, mit mindestens einer starken Säure, die in Wasser, bei 20 °C, einen pKₐ-Wert von weniger als 0,1 besitzt;
b) Destillation der Mischung, die in Schritt a) erhalten wurde;
c) Rückgewinnung des Destillats des Schrittes b), welches im Wesentlichen aus der Fluorcarbonsäure besteht.

2. Verfahren nach Anspruch 1, **dadurch gekennzeichnet, dass** die starke Säure dem wasserhaltigen Stoffstrom zugesetzt wird.

3. Verfahren nach Anspruch 1, **dadurch gekennzeichnet, dass** der wasserhaltige Stoffstrom der starken Säure zugesetzt wird.

4. Verfahren nach einem beliebigen der vorhergehenden Ansprüche, **dadurch gekennzeichnet, dass** der wasserhaltige Stoffstrom 5 bis 70 Gew.-%, vorzugsweise 10 bis 50 Gew.-%, mindestens einer Fluorcarbonsäure umfasst, bezogen auf das Gesamtgewicht des Stoffstroms.

5. Verfahren nach einem beliebigen der vorhergehenden Ansprüche, **dadurch gekennzeichnet, dass** der wasserhaltige Stoffstrom 0 bis 20 Gew.-%, vorzugsweise 0 bis 15 Gew.-%, an Verunreinigungen umfasst, bezogen auf das Fluorcarbonsäure(n)gewicht.

6. Verfahren nach einem beliebigen der vorhergehenden Ansprüche, **dadurch gekennzeichnet, dass** mindestens eine Fluorcarbonsäure aus den aliphatischen Fluorcarbonsäuren, die mit Wasser ein Azeotrop bilden, ausgewählt ist, vorzugsweise aus denjenigen, die eine aliphatische, lineare oder verzweigte Kette aufweisen, welche insgesamt 1 bis 10 Kohlenstoffatome besitzt.

7. Verfahren nach einem beliebigen der vorhergehenden Ansprüche, **dadurch gekennzeichnet, dass** mindestens eine Fluorcarbonsäure aus den Perfluorcarbonsäuren und den Fluorcarbonsäuren ausgewählt ist, bei denen sämtliche Fluoratome ausnahmslos an demjenigen Kohlenstoffatom sitzen, welches sich in ω-Stellung zur Carbonsäuregruppe befindet.

8. Verfahren nach einem beliebigen der vorhergehenden Ansprüche, **dadurch gekennzeichnet, dass** mindestens eine Fluorcarbonsäure aus Difluoressigsäure, Chlordifluoressigsäure, Trifluoressigsäure, 3,3,3-Trifluorpropionsäure, Pentafluorpropionsäure, Heptafluorbuttersäure, Perfluorpentansäure, Perfluorhexansäure, Perfluorheptansäure und Perfluoroctansäure ausgewählt ist.

9. Verfahren nach einem beliebigen der vorhergehenden Ansprüche, **dadurch gekennzeichnet, dass** es sich bei mindestens einer Fluorcarbonsäure um Trifluoressigsäure handelt.

10. Verfahren nach einem beliebigen der vorhergehenden Ansprüche, **dadurch gekennzeichnet, dass** die starke Säure, bei 20 °C in Wasser, einen pKₐ-Wert von weniger als -1 besitzt.

11. Verfahren nach einem beliebigen der vorhergehenden Ansprüche, **dadurch gekennzeichnet, dass** die starke Säure aus Schwefelsäure, den Oleum-Formen (mit verschiedenen Konzentrationen, 20 %, 30 % oder 40 %), Salzsäure in flüssigem oder gasförmigem Zustand, Phosphorsäure, Chlorsulfonsäure, Fluorsulfonsäure, Perchlorsäure, den Sulfonsäuren, vorzugsweise den Methan-, Trifluormethan-, Toluol- oder Phenolsulfonsäuren, den geträgerten Säuren wie, unter anderem, den Harzen mit Sulfonsäuregruppen, den sulfonsäurehaltigen Styrol-Divinylbenzol-Copolymeren, den Harzen mit Sulfonsäuregruppen, welche Phenol-Formaldehyd-Copolymere darstellen und am aromatischen Kern eine Methylensulfonsäuregruppe tragen, den perfluorierten Harzen, welche Sulfonsäuregruppen tragen, den Copolymeren aus Tetrafluorethylen und Perfluor-[2-(fluorsulfonylethoxy)propyl]vinylether sowie den Mischungen aus zweien oder mehreren davon in beliebigen Anteilen ausgewählt ist.

12. Verfahren nach einem beliebigen der vorhergehenden Ansprüche, **dadurch gekennzeichnet, dass** es sich bei der starken Säure um Schwefelsäure, vorzugsweise um Schwefelsäure mit 98 Gew.-% handelt.

13. Verfahren nach einem beliebigen der vorhergehenden Ansprüche, **dadurch gekennzeichnet, dass** das Gewichtsverhältnis von Säure(n) / im Stoffstrom enthaltenem Wasser im Bereich von 1 bis 10, vorzugsweise von 1,5 bis 3, liegt.

14. Verfahren nach einem beliebigen der vorhergehenden Ansprüche, **dadurch gekennzeichnet, dass** der Destillationsschritt bei Atmosphärendruck durchgeführt wird.

15. Verfahren nach einem beliebigen der vorhergehenden Ansprüche, **dadurch gekennzeichnet, dass** die Fluorcarbonsäure mit einem Reinheitsgrad von mindestens 95 Gew.-%, vorzugsweise von mindestens 99 Gew.-%, zurückgewonnen wird.

16. Verfahren nach einem beliebigen der vorhergehenden Ansprüche, **dadurch gekennzeichnet, dass** die Ausbeute der Rückgewinnung mehr als 50 Gew.-%, vorzugsweise mehr als 75 Gew.-%, mit noch größerem Vorzug mehr als 90 Gew.-%, oder sogar mehr als 95 Gew.-% beträgt.

17. Verfahren nach einem beliebigen der vorhergehenden Ansprüche, **dadurch gekennzeichnet, dass** der Destillationsschritt chargenweise (batch) durchgeführt wird.

18. Verfahren nach einem der Ansprüche 1 bis 16, **dadurch gekennzeichnet, dass** der Destillationsschritt kontinuierlich durchgeführt wird.

19. Verwendung einer Destillationsvorrichtung zur Durchführung des Verfahrens, das in einem der Ansprüche 1 bis 18 beschrieben ist, wobei die Vorrichtung Folgendes umfasst:
- einen Reaktor (Kessel), der ein Reaktionsmilieu enthält, welches einen wasserhaltigen Stoffstrom umfasst, der 5 bis 70 Gew.-%, vorzugsweise 10 bis 50 Gew.-%, mindestens einer Fluorcarbonsäure, bezogen auf das Gesamtgewicht des Stoffstroms, und mindestens eine starke Säure umfasst, wobei das Gewichtsverhältnis von Säure(n) / im Stoffstrom enthaltenem Wasser im Bereich von 1 bis 10 liegt;
- Erhitzungsmittel;
- Mittel zur Destillation und Kondensation der Dämpfe, die aus der Reaktionsmischung stammen, welche unter Rückfluss erhitzt wird;
- Mittel zum Messen und/oder Steuern der Temperatur, und möglicherweise Mittel, die das Rühren der Reaktionsmischung ermöglichen.
